# NOUVEAU FASCICULE DE BREVET EUROPEEN

(11) **EP 0 465 340 B2**
(45) Date de publication et mention de la décision concernant l'opposition: **05.03.1997**
(45) Mention de la délivrance du brevet: 29.12.1993
(21) Numéro de dépôt: 91401802.3
(22) Date de dépôt: 02.07.1991
(51) Int. Cl.: A61K 7/13

(54) **Procédé de teinture des fibres kératiniques avec le 4-hydroxyindole à pH acide et compositions**
Verfahren zur keratinischen Färbung mit 4-Hydroxyindol bei säurigem pH-Wert und Mittel
Keratinous fibres dyeing process with 4-hydroxyindol at acidic pH and compositions

(30) Priorité: 05.07.1990 FR 9008569
(43) Date de publication de la demande: 08.01.1992
(73) Titulaire: L'OREAL, 75008 Paris (FR)
(72) Inventeur: Cotteret, Jean, F-78480 Verneuil-sur-Seine (FR)
(74) Mandataire: Casalonga, Axel

(56) Documents cités:
- EP-A- 0 271 186
- EP-A- 0 360 638
- DE-A- 3 031 709
- DE-A- 3 842 508
- DE-A- 3 930 474
- FR-A- 2 636 235
- GB-A- 2 211 517
- Derwent Ref. 86-141936/22 & JP A 61-78 714

## Description

La présente invention est relative à un procédé de teinture des fibres kératiniques, en particulier des fibres kératiniques humaines, mettant en oeuvre le 4-hydroxyindole en association avec des bases d'oxydation et un agent oxydant, en milieu acide et aux compositions mises en oeuvre au cours de ce procédé.

Il est connu de teindre les fibres kératiniques et en particulier les cheveux humains avec des compositions tinctoriales contenant, en milieu alcalin, des précurseurs de colorants d'oxydation et en particulier des p-phénylènediamines, des ortho ou para-aminophénols appelés généralement "bases d'oxydation".

On sait également que l'on peut faire varier les nuances obtenues avec ces bases d'oxydation en les associant à des coupleurs encore appelés modificateurs de coloration, choisis notamment parmi les métadiamines aromatiques, les métaaminophénols et les métadiphénols.

L'utilisation de coupleurs pour bleus en teinture d'oxydation est particulièrement intéressante pour la réalisation de nuances naturelles ou cendrées. Dans le cadre habituel des teintures par oxydation en milieu alcalin, on a déjà proposé l'utilisation comme coupleurs pour bleus des dérivés métaphénylènediamines ou bien des composés indoliques comme le 4-hydroxyindole, en présence de bases d'oxydation telles que la paraphénylènediamine.

La demanderesse vient de découvrir d'une manière surprenante, que l'utilisation, comme coupleur, du 4-hydroxyindole en association avec des bases d'oxydation en un mélange extemporané avec un oxydant, à un pH acide, permettait d'obtenir des colorations naturelles et cendrées présentant une puissance tinctoriale améliorée et une remarquable ténacité aux shampooings, à la transpiration, aux traitements chimiques et aux agents atmosphériques tels que la lumière.

La présente invention a donc pour objet un procédé de teinture des fibres kératiniques, en particulier des fibres kératiniques humaines telles que les cheveux, comprenant l'application sur ces fibres d'au moins une composition contenant le 4-hydroxyindole comme coupleur, au moins un précurseur de colorant d'oxydation et un agent oxydant, à pH acide.

L'invention a également pour objet un agent de teinture à deux composants, dont l'un des composants comprend le 4-hydroxyindole et au moins un précurseur de colorant d'oxydation et l'autre l'agent oxydant à un pH acide, en des quantités telles que le mélange présente un pH acide.

L'invention a également pour objet la composition prête à l'emploi, contenant les différents agents utilisés pour la teinture en milieu acide, des cheveux.

D'autre objets de l'invention apparaîtront à la lecture de la description et des exemples qui suivent.

Le procédé de teinture des fibres kératiniques et en particulier des fibres kératiniques humaines telles que les cheveux, conforme à l'invention, est essentiellement caractérisé par le fait que l'on applique sur ces fibres une composition contenant dans un milieu approprié pour la teinture :
- au moins le 4-hydroxyindole comme coupleur, ainsi que les sels de ce composé;
- au moins un précurseur de colorant d'oxydation;
- au moins un agent oxydant; le pH de la composition appliquée sur les fibres étant inférieur à 7.

Les précurseurs de colorants d'oxydation ou bases d'oxydation sont des composés connus qui ne sont pas des colorants en eux-mêmes et qui forment un colorant par un processus de condensation oxydative, soit sur eux-mêmes, soit en présence d'un coupleur ou modificateur. Ces composés comportent généralement un noyau aromatique portant des groupements fonctionnels, constitués : soit par deux groupements amino; soit par un groupement amino et un groupement hydroxy; ces groupements étant en position para ou ortho, l'un par rapport à l'autre.

Les précurseurs de colorants d'oxydation de type para, utilisés conformément à l'invention, sont choisis plus particulièrement parmi les paraphénylène diamines, les para-aminophénols, les précurseurs hétérocycliques para, comme la 2,5-diaminopyridine, la 2-hydroxy 5-aminopyridine, la 2,4,5,6-tétraamino pyrimidine.

Parmi les paraphénylènediamines, on peut citer les composés répondant à la formule (I) : 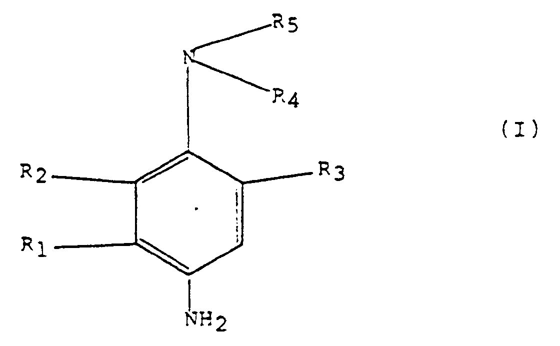 dans laquelle R₁, R₂ et R₃, identiques ou différents, représentent un atome d'hydrogène ou d'halogène, un radical alkyle ayant de 1 à 4 atomes de carbone, un radical alcoxy ayant de 1 à 4 atomes de carbone; R₄ et R₅, identiques ou différents, représentent un atome d'hydrogène, un radical alkyle, hydroxyalkyle, alcoxy alkyle, carbamylalkyle, mésylaminoalkyle, acétylamino alkyle, uréidoalkyle, carbalcoxyaminoalkyle, pipéridino alkyle, morpholinoalkyle; ces groupes alkyle ou alcoxy ayant de 1 à 4 atomes de carbone, ou bien R₄ et R₅ forment, conjointement avec l'atome d'azote auquel ils sont liés, un hétérocycle pipéridino ou morpholino, sous réserve que R₁ ou R₃ représente un atome d'hydrogène lorsque R₄ et R₅ ne représentent pas un atome d'hydrogène, ainsi que les sels de ces composés.

Parmi les composés particulièrement préférés répondant à la formule (I), on peut citer la p-phénylènediamine, la 2-méthyl-p-phénylènediamine, la 2,6-diméthylparaphénylénediamine, la 2,5-diméthyl paraphénylènediamine, la 2,3-diméthylparaphénylène diamine, la méthoxyparaphénylènediamine, la chloroparaphénylènediamine, la 2-méhtyl 5-méthoxypara phénylénediamine, la 2,6-diméthyl 5-méthoxyparaphénylène diamine, la N,N-diméthylparaphénylènediamine, la 3-méthyl 4-amino N,N-diéthylaniline, la N,N-di-(β-hydroxyéthyl)paraphénylènediamine, la 3-méthyl 4-amino N,N-di(β-hydroxyéthyl)aniline, la 3-chloro 4-amino N,N-di(β-hydroxyéthyl)aniline, la 4-amino N,N-(éthyl, carbamylméthyl)aniline, la 3-méthyl 4-amino N,N-(éthyl, carbamylméthyl)aniline, la 4-amino N,N-(éthyl, β-pipéridinoéthyl)aniline, la 3-méthyl 4-amino N,N-(éthyl, β-pipéridinoéthyl)aniline, la 4-amino N,N-(éthyl, β-morpholinoéthyl)aniline, la 3-méthyl 4-amino N,N-(éthyl, β-morpholinoéthyl)aniline, la 4-amino N,N-(éthyl, β-acétylaminoéthyl)aniline, la 4-amino N-(β-méthoxyéthyl)aniline, la 3-méthyl 4-amino N,N-(éthyl, β-acétylamino éthyl)aniline, la 4-amino N,N-(éthyl, β-mésylaminoéthyl)aniline, la 3-méthyl 4-amino N,N-(éthyl, β-mésylamino éthyl)aniline, la 4-amino N,N-(éthyl, β-sulfoéthyl)aniline, la 3-méthyl 4-amino N,N-(éthyl, β-sulfoéthyl)aniline, la N-[(4'-amino)phényl]morpholine, la N-[(4'-amino)phényl]pipéridine.

Ces précurseurs de colorants par oxydation de type para peuvent être introduits dans la composition tinctoriale, soit sous forme de base libre, soit sous forme de sels, tels que sous forme de chlorhydrate, de bromhydrate ou de sulfate.

Parmi les p-aminophénols, on peut citer le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2-hydroxyméthyl 4-aminophénol, le 2-(β-hydroxyéthyl) 4-aminophénol, le 2-méthoxy 4-aminophénol, le 3-méthoxy 4-aminophénol, le 2,5-diméthyl 4-aminophénol, le 2-méthoxyméthyl 4-aminophénol.

Les précurseurs de colorants d'oxydation de type ortho sont choisis parmi les orthoaminophénols, comme le 1-amino 2-hydroxybenzène, le 6-méthyl 1-hydroxy 2-aminobenzène, le 4-méthyl 1-amino 2-hydroxybenzène et les orthophénylènediamines.

Les précurseurs de colorants d'oxydation utilisés tout particulièrement selon la présente invention, sont :
- la paraphénylènediamine
- la 2-méthylparaphénylènediamine
- la 2,6-diméthylparaphénylènediamine
- le para-aminophénol
- la chloroparaphénylènediamine.

L'agent oxydant est choisi de préférence parmi le peroxyde d'hydrogène, le peroxyde d'urée, les bromates de métaux alcalins, les persels tels que les perborates et les persulfates. Le peroxyde d'hydrogène est particulièrement préféré.

Les compositions tinctoriales ne contiennent pas d'ions iodures dans des quantités suffisantes pour oxyder le 4-hydroxyindole et le précurseur de colorant d'oxydation.

Le pH de la composition appliquée sur les fibres kératiniques, en particulier les cheveux, a une valeur inférieure à 7 et est compris de préférence entre 3 et 6,9. Ce pH est ajusté par l'utilisation d'agents acidifiants bien connus dans le domaine de la teinture des fibres kératiniques, et en particulier des cheveux humains, tels que des acides minéraux comme l'acide chlorhydrique ou l'acide phosphorique, ou organiques comme les acides carboxyliques tels que l'acide tartrique ou citrique.

Le 4-hydroxyindole est présent dans la composition appliquée sur les fibres kératiniques, dans des proportions comprises de préférence entre 0,01 et 3,5% en poids par rapport au poids total de la composition.

Les compositions définies ci-dessus appliquées dans la teinture des fibres kératiniques peuvent également contenir en plus du 4-hydroxyindole, d'autres coupleurs connus en eux-mêmes tels que des métadiphénols, des métaaminophénols, des métaphénylène diamines, des méta N-acylaminophénols, des métauréidophénols, des métacarbalcoxyaminophénols, l'α-naphtol, des coupleurs possédant un groupement méthylène actif, tels que les composés dicétoniques, les pyrazolones.

Parmi ces coupleurs pouvant être utilisés en plus du 4-hydroxyindole, on peut citer le 2,4-dihydroxyphénoxyéthanol, le 2,4-dihydroxyanisole, le métaaminophénol, la résorcine, le monométhyléther de résorcine, la 2-méthylrésorcine, le pyrocatéchol, le 2-méthyl 5-N-(β-hydroxyéthyl)aminophénol, le 2-méthyl 5-N-(β-mésylaminoéthyl)aminophénol, la 6-hydroxybenzo morpholine, le 2,4-diaminoanisole, le 2,4-diamino phénoxyéthanol, la 6-aminobenzomorpholine, le [2-N-(β-hydroxyéthyl)amino 4-amino]-phénoxyéthanol, le 2-amino 4-N-(β-hydroxyéthyl)aminoanisole, le (2,4-diamino)phényl-β,γ-dihydroxypropyléther, la 2,4-diamino phénoxyéthylamine, le 1,3-diméthoxy 2,4-diaminobenzène, le 2-méthyl 5-aminophénol, le 2,6-diméthyl 3-amino phénol, le 3,4-méthylènedioxyphénol et la 3,4-méthylène dioxyaniline et leurs sels.

Les compositions définies ci-dessus peuvent également contenir des colorants direct tels que les dérivés nitrés de la série benzénique, les azoïques, les anthraquinoniques, les triphénylméthanes, les colorants xanthéniques ou azéniques bien connus du technicien. Elles peuvent également contenir des colorants d'oxydation rapides.

Ces compositions peuvent également contenir des agents tensio-actifs anioniques, cationiques, non ioniques, amphotères ou leurs mélanges.

Parmi ces agents tensio-actifs, on peut citer les alkylbenzènesulfonates, les alkylnaphthalène sulfonates, les sulfates, les éthersulfates et les sulfonates d'alcools gras, les sels d'ammonium quaternaires tels que le bromure de triméthylcétyl ammonium, le bromure de cétylpyridinium, les éthanolamides d'acides gras éventuellement oxyéthylénés, les acides, les alcools ou les amines polyoxyéthylénés, les alcools polyglycérolés, les alkylphénols polyoxyéthylénés ou polyglycérolés, ainsi que les alkylsulfates polyoxyéthylénés.

Les compositions tinctoriales sont généralement aqueuses, mais elles peuvent également contenir des solvants organiques pour solubiliser des composés qui ne seraient pas suffisamment solubles dans l'eau. Parmi ces solvants, on peut citer à titre d'exemple, les alcanols inférieurs en C₂-C₄ tels que l'éthanol et l'isopropanol, le glycérol, les glycols ou éthers de glycol, comme le butoxy-2 éthanol, l'éthylène glycol, le propylèneglycol, le monoéthyléther et le monométhyléther du diéthylèneglycol, le monoéthyléther et le monométhyléther du propylèneglycol, ainsi que les alcools aromatiques comme l'alcool benzylique ou le pnénoxyéthanol, ou les mélanges de ces solvants.

La composition appliquée sur les cheveux peut également renfermer des agents épaississants choisis en particulier parmi l'alginate de sodium, la gomme arabique, les dérivés de cellulose tels que la méthylcellulose, l'hydroxyéthycellulose, l'hydroxy propycellulose, l'hydroxyméthylcellulose, la carboxyméthylcellulose, les polymères d'acide acrylique éventuellement réticulés, la gomme de xanthane. On peut également utiliser des agents épaississants minéraux tels que la bentonite.

La composition peut également renfermer les agents antioxydants choisis en particulier parmi le sulfite de sodium, l'acide thioglycolique, le bisulfite de sodium, l'acide ascorbique et l'hydroquinone, ainsi que d'autres adjuvants cosmétiquement acceptables lorsque la composition est destinée à être utilisée pour la teinture des fibre kératiniques humaines, tels que des agents de pénétration, des agents séquestrants, des conservateurs, des tampons, des parfums, etc...

La composition appliquée sur les cheveux peut se présenter sous des formes diverses, telles que de liquides, de crèmes, de gels ou sous toute autre forme appropriée pour réaliser une teinture capillaire. Elle peut être conditionnée en flacon aérosol en présence d'un agent propulseur.

L'invention a également pour objet la composition prête à l'emploi utilisée dans le procédé défini ci-dessus.

Selon une forme de réalisation préférée, le procédé comporte une étape préliminaire consistant à stocker sous forme séparée, d'une part, la composition contenant dans un milieu approprié pour la teinture le coupleur 4-hydroxyindole et les précurseurs de colorants par oxydation sous forme d'un composant (A) et, d'autre part, une composition renfermant l'agent oxydant tel que défini ci-dessus sous forme d'un composant (B), et à procéder à leur mélange extemporané avant d'appliquer ce mélange sur les fibres kératiniques, comme indiqué ci-dessus.

La composition appliquée sur les fibres kératiniques résulte d'un mélange de 10 à 90% du composant (A) avec 90 à 10% du composant (B) contenant un agent oxydant.

L'invention a également pour objet un agent de teinture des fibres kératiniques, en particulier des cheveux, essentiellement caractérisé par le fait qu'il comporte au moins deux composants, l'un des composants étant constitué par le composant (A) défini ci-dessus et l'autre étant constitué par le composant (B) également défini ci-dessus, le pH des composants (A) et (B) étant tel qu'après mélange dans des proportions de 90 à 10% pour le composant (A) et de 10 à 90% pour le composant (B), la composition résultante ait un pH inférieur à 7.

Dans cette forme de réalisation, le composant (A) qui renferme au moins le 4-hydroxyindole et un précurseur de colorant d'oxydation, peut avoir un pH compris entre 3 et 10,5 et peut être ajusté à la valeur choisie au moyen d'agents alcalinisants habituellement utilisés en teinture des fibres kératiniques, tels que l'ammoniaque, les carbonates alcalins, les alcanolamines tels que les mono-, di- et triéthanolamines ainsi que leurs dérivés ou des agents acidifiants classiques, tels que les acides minéraux comme l'acide chlorhydrique ou l'acide phosphorique, ou organiques comme les acides carboxyliques tels que l'acide tartrique ou citrique.

Cette composition ne contient pas d'ions iodures dans des quantités suffisantes pour oxyder le 4-hydroxyindole et le précurseur de colorant d'oxydation.

Cette composition peut renfermer les différents autres adjuvants mentionés ci-dessus, notamment des coupleurs différents du 4-hydroxyindole.

L'ensemble des précurseurs de colorants par oxydation, de type para et/ou ortho ainsi que les coupleurs, sont présents dans des proportions comprises de préférence entre 0,05 et 7% en poids par rapport au poids total du composant (A). La concentration en 4-hydroxyindole peut varier entre 0,01 et 4% en poids par rapport au poids total du composant (A).

Les agents tensio-actifs sont présents dans le composant (A) dans des proportions de 0,1 à 55% en poids. Lorsque le milieu contient des solvants en plus de l'eau, ceux-ci sont présents dans des proportions comprises entre 0,5 et 40% en poids et en particulier entre 5 et 30% en poids par rapport au poids total du composant (A). Les agents épaississants sont présents de préférence dans des proportions comprises entre 0,1 et 5%, et en particulier entre 0,2 et 3% en poids. Les agents anti-oxydants mentionés ci-dessus sont de préférence présents dans le composant (A) dans des proportions comprises entre 0,02 et 1,5% en poids par rapport au poids total du composant (A).

Le composant (B) renfermant l'agent oxydant tel que défini ci-dessus, a un pH inférieur à 7. Ce pH peut avoir une valeur minimum de 1 et de préférence inférieur à 5. Ce composant (B) peut être acidifié avec le même type d'agents acidifiants que ceux utilisés pour le composant (A).

Il peut se présenter sous forme de liquide plus ou moins épaissi, de lait ou de gel.

Cet agent de teinture à deux composants peut être conditionné dans un dispositif à plusieurs compartiments ou kit de teinture, ou tout autre système de conditionnement à plusieurs compartiments dont l'un renferme le composant (A) et le second renferme le composant (B); ces dispositifs pouvant être équipés d'un moyen permettant de délivrer sur les cheveux le mélange souhaité, tel que les dispositifs décrits dans le brevet US-A-4 823 985 de la demanderesse.

L'invention a également pour objet l'utilisation comme coupleur du 4-hydroxyindole pour la teinture en milieu acide des fibres kératiniques, en association avec des précurseurs de colorants d'oxydation.

Conformément à l'invention, le procédé de teinture consiste à appliquer sur les cheveux le mélange obtenu, à le laisser poser pendant 3 à 40 minutes, puis à rincer les cheveux et éventuellement effectuer un shampooing.

Il est également possible, conformément à l'invention, d'appliquer séparément une composition contenant le coupleur 4-hydroxyindole, le précurseur de colorant d'oxydation et l'agent oxydant, de façon à ce que le mélange se formant in-situ au niveau des fibres ait un pH inférieur à 7, comme défini ci-dessus.

Les exemples suivants sont destinés à illustrer l'invention sans pour autant présenter un caractère limitatif.

### EXEMPLES 1 à 6

On procède à la teinture des cheveux en appliquant sur des cheveux gris à 90% de blancs, un mélange extemporané de la composition colorante (A) et de la composition oxydante (B).

Ce mélange présente le pH indiqué dans le tableau des exemples qui suivent. On laisse agir ce mélange pendant 30 minutes, puis on rince les cheveux, on effectue un shampooing. Après séchage, les cheveux sont teints dans la nuance précisée au bas du tableau ci-après.

| en g | 1 | 2 | 3 |
|---|---|---|---|
| A) Composition colorante | | | |
| 4-hydroxyindole | 0,399 | 0,133 | 0,266 |
| 2,6-diméthylparaphénylènediamine, 2HCl | 0,627 | | |
| 2,3-diméthylparaphénylènediamine, 2HCl | | | 0,418 |
| 2-méthylparaphénylènediamine, 2HCl | | 0,366 | |
| Métaaminophénol | | 0,109 | |
| α-naphtol | | 0,144 | |
| Monoéthanolamine qs pH | 8,9 | 9,0 | 9,0 |
| Support 2 | X | X | X |
| Eau qsp | 100 | 100 | 100 |

| B) Composition oxydante | | | |
|---|---|---|---|
| Solution d'eau oxygénée à 20 volumes | | | |
| Acide phosphorique qs pH | 1,2 | 1,2 | 1,2 |
| pH mélange p/p A + B | 6,3 | 6,5 | 6,5 |
| Nuances obtenues : | violet | violet cendré nacré | bleu cendré léger |

| | 4 | 5 | 6 |
|---|---|---|---|
| A) Composition colorante | | | |
| 4-hydroxyindole | 0,266 | 0,399 | 0,399 |
| Paraphénylènediamine | | 0,324 | |
| Paraaminophénol | 0,436 | | 0,327 |
| 2-méthyl 5-N-(β-hydroxyéthyl)aminophénol | 0,334 | | |
| Monoéthanolamine qs pH | 9,9 | 9,8 | 9,8 |
| Support 1 | X | | |
| Support 3 | | X | X |
| Eau qsp | 100 | 100 | 100 |

| B) Composition oxydante | | | |
|---|---|---|---|
| Solution d'eau oxygénée à 20 volumes | | | |
| Acide phosphorique qs pH | 1,2 | 1,5 | 1,5 |
| pH mélange p/p A + B | | 5,5 | 5,5 |
| pH mélange 1/3 A + 2/3 B | 5,0 | | |
| Nuances obtenues : | blond clair cendré | cendré nacré | châtain |

### EXEMPLE 7

### COMPOSITION COLORANTE :

- 2,5-diaminonitrobenzène 0,3 g
- 4-hydraxyindole 0,25 g
- Paraphénylènediamine 0,4 g
- Métaaminophénol 0,15 g
- Lauryléthersulfate de sodium à 2 moles d'oxyde d'éthylène, vendu à 28 % de MA 4,2 g MA
- Nonylphénol oxyéthyléné à 9 moles d'oxyde d'éthylène 1,0 g
- Monobutyléther d'éthylèneglycol 9,5 g
- Métabisulfite de sodium à 35% de MA 0,45 g MA
- Monoéthanolamine qs pH=8,4
- Séquestrant qs
- Eau qsp 100 g

Mélange poids pour poids avec composition oxydante : solution d'eau oxygénée à 20 volumes dont le pH est ajusté à 1,3 par l'acide phosphorique.

pH spontané du mélange : 6,3.

Application : comme dans les exemples précédents 1 à 6.

On obtient sur des cheveux gris naturels à 90% de blancs, une coloration châtain acajou irisé.

### SUPPORT DE COLORATION 1

- Nonylphénol à 4 moles d'oxyde d'éthylène, vendu sous la dénomination SINNOPAL NP4 par la Société HENKEL 25,5 g
- Nonylphénol à 9 moles d'oxyde d'éthylène, vendu sous la dénomination SINNOPAL NP9 par la Société HENKEL 17,5 g
- Monoéthyléther d'éthylèneglycol 7,0 g
- Propyléneglycol 10,5 g
- Dipropylèneglycol 0,5 g
- Alcool éthylique 2,0 g
- Lauryléther sulfate de monoéthanolamine, vendu sous la dénomination SACTIPON 2 OM 29 par la Société LEVER à 28% de MA 4,2 g MA
- Alkyléther sulfate de sodium à 28% de MA 0,8 g MA
- Métabisulfite de sodium en solution aqueuse à 35% de MA 0,45 g MA
- Acétate de sodium 0,8 g
- Antioxydant, séquestrant qs

### SUPPORT DE COLORATION 2

- Alcool oléique polyglycérolé à 2 moles de glycérol 4,0 g
- Alcool oléique polyglycérolé à 4 moles de glycérol à 78% de MA 5,69 g MA
- Acide oléique 3,0 g
- Amine oléique à 2 moles d'oxyde d'éthylène, vendue sous la dénomination ETHOMEEN O 12 par la Société AKZO 7,0 g
- Laurylaminosuccinamate de diéthylaminopropyle, sel de sodium à 55% de MA 3,0 g MA
- Alcool oléique 5,0 g
- Diéthanolamide d'acide oléique 12,0 g
- Propylèneglycol 3,5 g
- Alcool éthylique 7,0 g
- Dipropyléneglycol 0,5 g
- Monométhyléther de propylèneglycol 9,0 g
- Métabisulfite de sodium en solution aqueuse à 35% de MA 0,45 g MA
- Acétate d'ammonium 0,8 g
- Antioxydant, séquestrant qs

### SUPPORT DE COLORATION 3

- Nonylphénol à 4 moles d'oxyde d'éthylène, vendu sous la dénomination SINNOPAL NP4 par la Société HENKEL 25,5 g
- Nonylphénol à 9 moles d'oxyde d'éthylène, vendu sous la dénomination SINNOPAL NP9 par la Société HENKEL 17,5 g
- Butyléther d'éthyléneglycol 7,0 g
- Propylèneglycol 11,0 g
- Alcool éthylique 2,0 g
- Lauryléther sulfate de monoéthanolamine, vendu sous la dénomination SACTIPON 2 OM 29 par la Société LEVER à 28% de MA 5,0 g MA
- Métabisulfite de sodium en solution aqueuse à 35% de MA 0,45 g MA
- Acétate de sodium 0,8 g
- Antioxydant, séquestrant qs

## Revendications

1. Procédé de teinture des fibres kératiniques, en particulier des fibres kératiniques humaines telles que les cheveux, caractérisé par le fait qu'on applique sur ces fibres une composition contenant dans un milieu approprié pour la teinture :
- au moins le 4-hydroxyindole comme coupleur, ainsi que les sels de ce composé;
- au moins un précurseur de colorant d'oxydation comportant un noyau aromatique portant deux groupements amino ou un groupement amino et un groupement hydroxy; les deux groupements etant en position para ou ortho l'un par rapport à l'autre;
- au moins un agent oxydant;
le pH de la composition appliquée sur les fibres étant inférieur à 7.

2. Procédé selon la revendication 1, caractérisé par le fait que les précurseurs de colorants d'oxydation sont choisis parmi les paraphénylène diamines, les para-aminophénols, les précurseurs hétérocycliques para.

3. Procédé selon la revendication 2, caractérisé par le fait que les paraphénylènediamines sont choisies parmi les composés répondant à la formule: 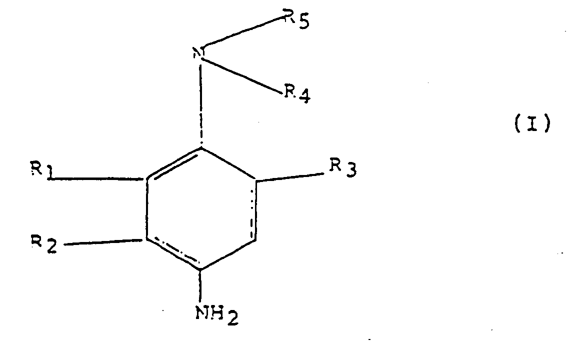 dans laquelles R₁, R₂ et R₃, identiques ou différents, représentent un atome d'hydrogène ou d'halogène, un radical alkyle ayant de 1 à 4 atomes de carbone, un radical alcoxy ayant de 1 à 4 atomes de carbone; R₄ et R₅, identiques ou différents, représentent un atome d'hydrogène, un radical alkyle, hydroxyalkyle, alcoxy alkyle, carbamylalkyle, mésylaminoalkyle, acétylamino alkyle, uréidoalkyle, carbalcoxyaminoalkyle, pipéridino alkyle, morpholinoalkyle; ces groupes alkyle ou alcoxy ayant de 1 à 4 atomes de carbone, ou bien R₄ et R₅ forment, conjointement avec l'atome d'azote auquel ils sont liés, un hétérocycle pipéridino ou morpholino, sous réserve que R₁ ou R₃ représente un atome d'hydrogène lorsque R₄ et R₅ ne représentent pas un atome d'hydrogène, ainsi que les sels de ces composés.

4. Procédé selon la revendication 3, carctérisé par le fait que les composés de formule (I) sont choisis parmi la p-phénylènediamine, la 2-méthyl-p-phénylènediamine, la méthoxyparaphénylène diamine, la chloroparaphénylènediamine, la 2,6-diméthylparaphénylènediamine, la 2,3-diméthylpara phénylènediamine, la 2,5-diméthylparaphénylènediamine, la 2-méthyl 5-méthoxyparaphénylènediamine, la 2,6-diméthyl 5-méthoxyparaphénylènediamine, la N,N-diméthylparaphénylènediamine, la 3-méthyl 4-amino N,N-diéthylaniline, la N,N-di-(β-hydroxyéthyl)para phénylènediamine, la 3-méthyl 4-amino N,N-di(β- hydroxyéthyl) aniline, la 3-chloro 4-amino N,N-di-(β-hydroxyéthyl)aniline, la 4-amino N,N-(éthyl,carbamyl méthyl)aniline, la 3-méthyl 4-amino N,N-(éthyl, carbamyl méthyl)aniline, la 4-amino N,N-(éthyl, β-pipéridinoéthyl)aniline, la 3-méthyl 4-amino N,N-(éthyl, β-pipéridino éthyl)aniline, la 4-amino N,N-(éthyl, β-morpholinoéthyl)aniline, la 3-méthyl 4-amino N,N-(éthyl, β-morpholino éthyl)aniline, la 4-amino N,N-(éthyl, β-acétylaminoéthyl)aniline, la 4-amino N-(β-méthoxyéthyl)aniline, la 3-méthyl 4-amino N,N-(éthyl, β-acétylaminoéthyl)aniline, la 4-amino N,N-(éthyl, β-mésylaminoéthyl)aniline, la 3-méthyl 4-amino N,N-(éthyl, β-mésylaminoéthyl)aniline, la 4-amino N,N-(éthyl, β-sulfoéthyl)aniline, la 3-méthyl 4-amino N,N-(éthyl, β-sulfoéthyl)aniline, la N-[(4'-amino)phényl]morpholine, la N-[(4'-amino)phényl] pipéridine sous forme de base libre ou de sels.

5. Procédé selon la revendication 2, caractérisé par le fait que les p-aminophénols sont choisis parmi le p- aminophénol, le 2-méthyl 4-amino phénol, le 3-méthyl 4-aminophénol, le 2-chloro 4-amino phénol, le 3-chloro 4-aminophénol, le 2,6-diméthyl 4-aminophénol, le 3,5-diméthyl 4-aminophénol, le 2,3-diméthyl 4-aminophénol, le 2-hydroxyméthyl 4-aminophénol, le 2-(β-hydroxyéthyl)4-aminophénol, le 2-méthoxy 4-aminophénol, le 3-méthoxy 4-aminophénol, le 2,5-diméthyl 4-aminophénol, le 2-méthoxyméthyl 4-aminophénol.

6. Procédé selon la revendication 1, caractérisé par le fait que les précurseurs de colorants d'oxydation sont des précurseurs de colorants d'oxydation de type ortho choisis parmi les ortho-aminophénols et les ortho-phénylénediamines.

7. Procédé selon l'une quelconque des revendications 1 à 6, caractérisé par le fait que l'agent oxydant est choisi parmi le peroxyde d'hydrogène, le peroxyde d'urée, les bromates de métaux alcalins, les persels.

8. Procédé selon l'une quelconque des revendications 1 à 7, caractérisé par le fait que le pH de la composition appliquée sur les fibres kératiniques est compris entre 3 et 6,9.

9. Procédé selon l'une quelconque des revendications 1 à 8, caractérisé par le fait que la composition utilisée pour la teinture des fibres kératiniques contient en plus du coupleur 4-hydroxy indole, d'autres coupleurs choisis parmi les métadiphénols, les métaaminophénols, les méta phénylènediamines, les méta N-acylaminophénols, les métauréidophénols, les métacarbalcoxyaminophénols, l'α-naphtol, les coupleurs possédant un groupement méthylène actif choisis parmi les composés dicétoniques et les pyrazolones.

10. Procédé selon la revendication 9, caractérisé par le fait que les coupleurs sont choisis parmi le 2,4-dihydroxyphénoxyéthanol, le 2,4-dihydroryanisole, le métaaminophénol, la résorcine, le monoéthyléther de résorcine, la 2-méthylrésorcine, le pyrocatéchol, le 2-méthyl 5-N-(β-hydroxyéthyl)amino phénol, le 2-méthyl 5-N-(β-mésylaminoéthyl)aminophénol, la 6-hydroxybenzomorpholine, le 2,4-diaminoanisole, le 2,4-diaminophénoxyéthanol, la 6-aminobenzomorpholine, le [2-N-(β-hydroxyéthyl)amino 4-amino]- phénoxyéthanol, le 2-amino 4-N-(β-hydroxyéthyl)aminoanisole, le (2,4-diamino)phényl-β,γ-dihydroxypropyléther, la 2,4-diaminophénoxyéthylamine, le 1,3-diméthoxy 2,4-diamino benzène, le 2-méthyl 5-aminophénol, le 2,6-diméthyl 3-aminophénol, le 3,4-méthylénedioxyphénol et la 3,4-méthylénedioxyaniline et leurs sels.

11. Procédé selon l'une quelconque des revendications 1 à 10, caractérisé par le fait que la composition contient en plus des colorants directs et/ou des colorants d'oxydation rapides.

12. Procédé selon l'une quelconque des revendications 1 à 11, caractérisé par le fait que la composition contient des agents tensio-actifs anioniques, cationiques, non ioniques, amphotères ou leurs mélanges; des agents épaississants, des agents anti-oxydants et/ou tout autre adjuvant cosmétiquement acceptable.

13. Procédé selon l'une quelconque des revendications 1 à 12, caractérisé par le fait que le milieu approprié pour la teinture est constitué par de l'eau ou un mélange d'eau et d'un solvant choisi parmi les alcanols inférieurs en C₂-C₄, le glycérol, les glycols, les éthers de glycols, les alcools aromatiques ou leurs mélanges.

14. Agent de teinture des fibres kératiniques et en particulier des cheveux, caractérisé par le fait qu'il comporte au moins deux composants; un composant (A) constitué par une composition contenant dans un milieu approprié pour la teinture, le coupleur 4-hydroxyindole et un précurseur de colorant d'oxydation tel que défini dans l'une quelconque des revendications 1 à 6, un composant (B) constitué par une composition contenant dans un milieu approprié pour la teinture, un agent oxydant, le pH des composants (A) et (B) étant tel qu'après mélange dans des proportions de 90 à 10% pour le composant (A) et de 10 à 90% pour le composant (B), la composition résultante ait un pH inférieur à 7.

15. Agent selon la revendication 14, caractérisé par le fait que le composant (A) a un pH compris entre 3 et 10,5.

16. Agent selon la revendication 14 ou 15, caractérisé par le fait que le composant (A) contient les précurseurs de colorants par oxydation du type para et/ou ortho tels que définis dans l'une quelconque des revendications 1 à 6, ainsi que les coupleurs tels que définis dans les revendications 1, 9 ou 10, dans des proportions comprises entre 0,05 et 7% en poids par rapport au poids total du composant (A).

17. Agent selon l'une quelconque des revendications 14 à 16, caractérisé par le fait que la concentration en 4-hydroxyindole est comprise entre 0,01 et 4% en poids par rapport au poids total du composant A.

18. Agent selon l'une quelconque des revendications 14 à 17, caractérisé par le fait que le composant (A) contient des agents tensio-actifs dans des proportions de 0,1 à 55% en poids, des agents solvants en plus de l'eau dans des proportions comprises entre 0,5 et 40% en poids, des agents épaississants dans des proportions comprises entre 0,1 et 5% en poids, des agents antioxydants dans des proportions comprises entre 0,02 et 1,5% en poids, et/ou tout autre adjuvant cosmétiquement acceptable.

19. Agent selon l'une quelconque des revendications 14 à 18, caractérisé par le fait que le composant (B) a un pH qui a une valeur minimum de 1 et inférieure à 7.

20. Procédé de teinture des fibres kératiniques et en particulier des cheveux, caractérisé par le fait qu'il comporte une première étape consistant à stocker sous forme séparée les composants (A) et (B) tels que définis dans l'une quelconque des revendications 14 à 19 et à procéder avant application au mélange des composants (A) et (B) dans des proportions de 10 à 90% pour le composant (A) et de 90 à 10% pour le composant (B), de façon à obtenir une composition ayant un pH inférieur à 7 et d'appliquer ce mélange immédiatement après préparation sur les fibres kératiniques.

21. Dispositif à plusieurs compartiments ou kit de teinture, caractérisé par le fait qu'il comporte au moins deux compartiments dont un premier compartiment renferme le composant (A) tel que défini dans l'une quelconque des revendications 14 à 18, et le second compartiment renferme le composant (B) tel que défini dans les revendications 14 et 19.

22. Dispositif selon la revendication 21, caractérisé par le fait qu'il est équipé d'un moyen permettant de délivrer sur les cheveux le mélange souhaité des composants (A) et (B).

23. Procédé de teinture selon l'une quelconque des revendications 1 à 13, caractérisé par le fait que l'on applique sur les cheveux la composition et qu'on la laisse poser pendant 3 à 40 minutes, que l'on rince les cheveux et qu'on procède éventuellement à un shampooing avant un nouveau rinçage et séchage.

24. Utilisation comme coupleur du 4-hydroxy indole pour la teinture en milieu acide des fibres kératiniques, en association avec des précurseurs de colorants d'oxydation comportant un noyau aromatique portant deux groupements amino ou un groupement amino et un groupement hydroxy; les deux groupements etant en position para ou ortho l'un par rapport à l'autre.

25. Composition de teinture de fibres kératiniques, prête à l'emploi telle que mise en oeuvre dans le procédé selon l'une quelconque des revendications 1 à 13.

26. Composition selon la revendication 25, contenant le composé 4-hydroxyindole dans des proportions de 0,01 à 3,5% en poids par rapport au poids total de la composition.

## Claims

1. Method for dyeing keratinous fibres, in particular human keratinous fibres such as hair, characterized in that a composition containing, in a medium appropriate for dyeing:
- at least 4-hydroxyindole as coupler, as well as the salts of this compound;
- at least one oxidation dye precursor comprising an aromatic ring carrying two amino groups or an amino group and a hydroxyl group; the two groups being in the para or ortho position relative to one another; and
- at least one oxidizing agent; is applied to said fibres, the pH of the composition applied to the fibres being lower than 7.

2. Method according to Claim 1, characterized in that the oxidation dye precursors are chosen from para-phenylenediamines, para-aminophenols and para heterocyclic precursors.

3. Method according to Claim 2, characterized in that the para-phenylenediamines are chosen from the compounds corresponding to the formula: 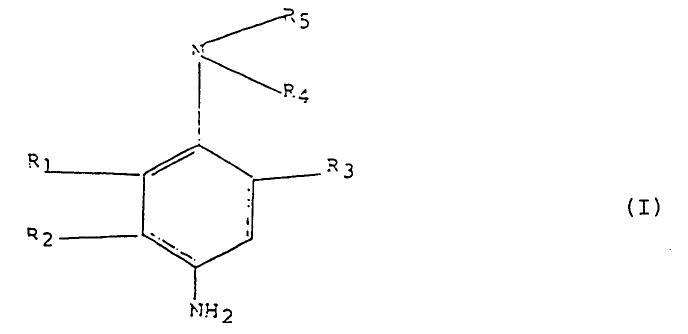 in which R₁, R₂ and R₃, which may be identical or different, represent a hydrogen or halogen atom, an alkyl radical having from 1 to 4 carbon atoms or an alkoxy radical having from 1 to 4 carbon atoms; and R₄ and R₅, which may be identical or different, represent a hydrogen atom or an alkyl, hydroxyalkyl, alkoxyalkyl, carbamylalkyl, mesylaminoalkyl, acetylaminoalkyl, ureidoalkyl, carbalkoxyaminoalkyl, piperidinoalkyl, or morpholinoalkyl radical; these alkyl or alkoxy groups having from 1 to 4 carbon atoms, or R₄ and R₅ form, together with the nitrogen atom to which they are bonded, a piperidino or morpholino heterocyclic ring, on condition that R₁ or R₃ represents a hydrogen atom when R₄ and R₅ do not represent a hydrogen atom, and the salts of these compounds.

4. Method according to Claim 3, characterized in that the compounds of formula (I) are chosen from p-phenylenediamine, 2-methyl-p-phenylenediamine, methoxy-para-phenylenediamine, chloro-para-phenylenediamine, 2,6-dimethyl-para-phenylenediamine, 2,3-dimethyl-para-phenylenediamine, 2,5-dimethyl-para-phenylenediamine, 2-methyl-5-methoxy-para-phenylenediamine, 2,6-dimethyl-5-methoxy-para-phenylenediamine, N,N-dimethyl-para-phenylenediamine, 3-methyl-4-amino-N,N-diethylaniline, N,N-di(β-hydroxyethyl)para-phenylenediamine, 3-methyl-4-amino-N,N-di(β-hydroxyethyl)aniline, 3-chloro-4-amino-N,N-di(β-hydroxyethyl)aniline, 4-amino-N,N-(ethyl, carbamylmethyl)aniline, 3-methyl-4-amino-N,N-(ethyl, carbamylmethyl)aniline, 4-amino-N,N-(ethyl,β-piperidinoethyl)aniline, 3-methyl-4-amino-N,N-(ethyl,β-piperidinoethyl)aniline, 4-amino-N,N-(ethyl,β-morpholinoethyl)aniline, 3-methyl-4-amino-N,N-(ethyl,β-morpholinoethyl)aniline, 4-amino-N,N-(ethyl,β-acetylaminoethyl)aniline, 4-amino-N-(β-methoxyethyl)aniline, 3-methyl-4-amino-N,N-(ethyl,β-acetylaminoethyl)aniline, 4-amino-N,N-(ethyl,β-mesylaminoethyi)aniline, 3-methyl-4-amino-N,N-(ethyl,β-mesylaminoethyl)aniline, 4-amino-N,N-(ethyl,β-sulphoethyl)aniline, 3-methyl-4-amino-N,N-(ethyl,β-sulphoethyl)aniline, N-[(4'-amino)phenyl]morpholine and N-[(4'-amino)phenyl]piperidine, in the form of the free base or of salts.

5. Method according to Claim 2, characterized in that the p-aminophenols are chosen from p-aminophenol, 2-methyl-4-aminophenol, 3-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol, 2-hydroxymethyl-4-aminophenol, 2-(β-hydroxyethyl)-4-aminophenol, 2-methoxy-4-aminophenol, 3-methoxy-4-aminophenol, 2,5-dimethyl-4-aminophenol and 2-methoxymethyl-4-aminophenol.

6. Method according to Claim 1, characterized in that the oxidation dye precursors are oxidation dye precursors of the ortho type chosen from ortho-aminophenols and ortho-phenylenediamines.

7. Method according to any one of Claims 1 to 6, characterized in that the oxidizing agent is chosen from hydrogen peroxide, urea peroxide, alkali metal bromates and persalts.

8. Method according to any one of Claims 1 to 7, characterized in that the pH of the composition applied to the keratinous fibres is between 3 and 6.9.

9. Method according to any one of Claims 1 to 8, characterized in that the composition used for dyeing keratinous fibres contains, in addition to the 4-hydroxyindole coupler, other couplers chosen from meta-diphenols, meta-aminophenols, meta-phenylenediamines, meta-N-acylaminophenols, meta-ureidophenols, meta-carbalkoxyaminophenols, α-naphthol and couplers containing an active methylene group chosen from diketone compounds and pyrazolones.

10. Method according to Claim 9, characterized in that the couplers are chosen from 2,4-dihydroxyphenoxyethanol, 2,4-dihydroxyanisole, meta-aminophenol, resorcinol, resorcinol monoethyl ether, 2-methylresorcinol, pyrocatechol, 2-methyl-5-N-(β-hydroxyethyl)aminophenol, 2-methyl-5-N-(β-mesylaminoethyl)aminophenol, 6-hydroxybenzomorpholine, 2,4-diaminoanisole, 2,4-diaminophenoxyethanol, 6-aminobenzomorpholine, [2-N-(β-hydroxyethyl)amino-4-amino]-phenoxyethanol, 2-amino-4-N-(β-hydroxyethyl)aminoanisole, (2,4-diamino)phenyl-β,γ-dihydroxypropyl ether, 2,4-diaminophenoxyethylamine, 1,3-dimethoxy-2,4-diaminobenzene, 2-methyl-5-aminophenol, 2,6-dimethyl-3-aminophenol, 3,4-methylenedioxyphenol and 3,4-methylenedioxyaniline and their salts.

11. Method according to any one of Claims 1 to 10, characterized in that the composition also contains direct dyes and/or rapid oxidation dyes.

12. Method according to any one of Claims 1 to 11, characterized in that the composition contains anionic, cationic, nonionic or amphoteric surface-active agents or their mixtures; thickeners, antioxidants and/or any other cosmetically acceptable adjuvant.

13. Method according to any one of Claims 1 to 12, characterized in that the medium appropriate for dyeing consists of water or a mixture of water and a solvent chosen from C₂-C₄ lower alkanols, glycerol, glycols, glycol ethers and aromatic alcohols or their mixtures.

14. Agent for dyeing keratinous fibres and in particular hair, characterized in that it comprises at least two components: a component (A) consisting of a composition containing, in a medium appropriate for dyeing, the 4-hydroxyindole coupler and an oxidation dye precursor as defined in any one of Claims 1 to 6, and a component (B) consisting of a composition containing an oxidizing agent in a medium appropriate for dyeing, the pH of the components (A) and (B) being such that, after mixing in proportions of 90 to 10 % in respect of component (A) and of 10 to 90 % in respect of component (B), the resulting composition has a pH lower than 7.

15. Agent according to Claim 14, characterized in that the component (A) has a pH of between 3 and 10.5.

16. Agent according to Claim 14 or 15, characterized in that the component (A) contains the oxidation dye precursors of the para and/or ortho type as defined in any one of Claims 1 to 6 and also the couplers as defined in Claims 1, 9 or 10 in proportions of between 0.05 and 7 % by weight relative to the total weight of the component (A).

17. Agent according to any one of Claims 14 to 16, characterized in that the 4-hydroxyindole concentration is between 0.01 and 4 % by weight relative to the total weight of the component (A).

18. Agent according to any one of Claims 14 to 17, characterized in that the component (A) contains surface-active agents in proportions of 0.1 to 55 % by weight, solvents in addition to water in proportions of between 0.5 and 40 % by weight, thickeners in proportions of between 0.1 and 5 % by weight, antioxidants in proportions of between 0.02 and 1.5 % by weight and/or any other cosmetically acceptable adjuvant.

19. Agent according to any one of Claims 14 to 18, characterized in that the component (B) has a pH which has a minimum value of 1 and is lower than 7.

20. Method for dyeing keratinous fibres and in particular hair, characterized in that it comprises a first step consisting in storing separately the components (A) and (B) as defined in any one of Claims 14 to 19 and, before application, in mixing the components (A) and (B) in proportions of 10 to 90 % in respect of component (A) and of 90 to 10 % in respect of component (B), so as to obtain a composition having a pH lower than 7, and applying this mixture immediately after preparation to the keratinous fibres.

21. Multi-compartment device or dyeing kit, characterized in that it comprises at least two compartments, a first compartment of which contains the component (A) as defined in any one of Claims 14 to 18 and the second compartment contains the component (B) as defined in Claims 14 and 19.

22. Device according to Claim 21, characterized in that it is fitted with means permitting the desired mixture of components (A) and (B) to be delivered onto the hair.

23. Dyeing method according to any one of Claims 1 to 13, characterized in that the composition is applied to the hair and in that it is left on the hair for 3 to 40 minutes, in that the hair is rinsed and in that shampooing is optionally carried out before rinsing again and drying.

24. Use of 4-hydroxyindole as a coupler for dyeing keratinous fibres in an acidic medium, in combination with oxidation dye precursors comprising an aromatic ring carrying two amino groups or an amino group and a hydroxyl group; the two groups being in the para or ortho position relative to one another.

25. Ready-to-use composition for dyeing keratinous fibres, as used in the method according to any one of Claims 1 to 13.

26. Composition according to Claim 25, containing the compound 4-hydroxyindole in proportions of from 0.01 to 3.5 % by weight relative to the total weight of the composition.

## Patentansprüche

1. Verfahren zur Färbung keratinischer Fasern, insbesondere menschlicher keratinischer Fasern wie der Haare,
dadurch **gekennzeichnet**, daß
man auf diese Fasern eine Zusammensetzung aufbringt, enthaltend in einem zur Färbung geeigneten Medium:
- mindestens 4-Hydroxyindol als Kuppler, sowie die Salze dieser Verbindung,
- mindestens eine Oxidationsfarbstoffvorstufe, die einen aromatischen Kern mit 2 Amino- oder 1 Amino- und 1 Hydroxi-Gruppen aufweist, wobei die beiden Gruppen in p- oder o-Position zueinander vorliegen, und
- mindestens ein Oxidationsmittel,
wobei der pH der auf die Fasern aufgetragenen Zusammensetzung weniger als 7 beträgt.

2. Verfahren gemäß Anspruch 1,
dadurch **gekennzeichnet**, daß
die Oxidationsfarbstoffvorstufen ausgewählt sind aus p-Phenylendiaminen, p-Aminophenolen, heterozyklischen p-Vorstufen.

3. Verfahren gemäß Anspruch 2,
dadurch **gekennzeichnet**, daß
die p-Phenylendiamine ausgewählt sind aus Verbindungen der Formel: 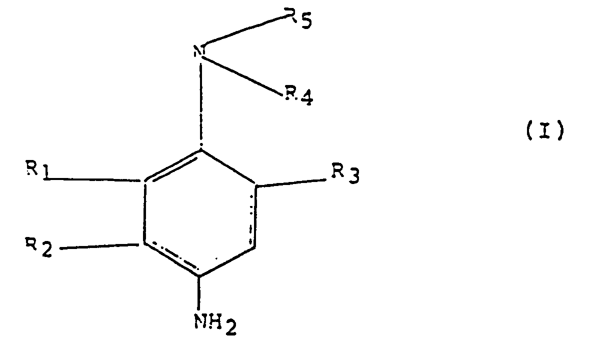 worin R₁, R₂ und R₃, gleich oder verschieden, ein Wasserstoff- oder Halogenatom, einen Alkylrest mit 1 bis 4 Kohlenstoffatomen, einen Alkoxyrest mit 1 bis 4 Kohlenstoffatomen, R₄ und R₅, gleich oder verschieden, ein Wasserstoffatom, einen Alkyl-, Hydroxyalkyl-, Alkoxyalkyl-, Carbamylalkyl-, Mesylaminoalkyl-, Acetylaminoalkyl-, Ureidoalkyl-, Carbalkoxyaminoalkyl-, Piperidinoalkyl- oder Morpholinoalkylrest darstellen, wobei diese Alkyl- oder Alkoxygruppen 1 bis 4 Kohlenstoffatome aufweisen, oder auch R₄ und R₅ zusammen mit dem Stickstoffatom, an das sie gebunden sind, einen Piperidino- oder Morpholinoheterozyklus bilden, mit der Maßgabe, daß R₁ oder R₃ ein Wasserstoffatom darstellen, wenn R₄ und R₅ kein Wasserstoffatom darstellen, sowie aus den Salzen dieser Verbindungen.

4. Verfahren gemäß Anspruch 3,
dadurch **gekennzeichnet**, daß
die Verbindungen der Formel (I) ausgewählt sind aus p-Phenylendiamin, 2-Methyl-p-phenylendiamin, Methoxy-p-phenylendiamin, Chlor-p-phenylendiamin, 2,6-Dimethyl-p-phenylendiamin, 2,3-Dimethyl-p-phenylendiamin, 2,5-Dimethyl-p-phenylendiamin, 2-Methyl-5-methoxy-p-phenylendiamin, 2,6-Dimethyl-5-methoxy-p-phenylendiamin, N,N-Dimethyl-p-phenylendiamin, 3-Methyl-4-amino-N,N-diethylanilin, N,N-Di(β-hydroxyethyl)-p-phenylendiamin, 3-Methyl-4-amino-N,N-di(β-hydroxyethyl)anilin, 3-Chlor-4-amino-N,N-di(β-hydroxyethyl)anilin, 4-Amino-N,N-(ethyl,carbamylmethyl)anilin, 3-Methyl-4-amino-N,N-(ethylcarbamylmethyl)anilin, 4-Amino-N,N-(ethyl,β-piperidinoethyl)anilin, 3-Methyl-4-amino-N,N-(ethyl-β-morpholinoethyl)anilin, 4-Amino-N,N-(ethyl-β-morpholinoethyl)anilin, 3-Methyl-4-amino-N,N-(ethyl-β-morpholinoethyl)anilin, 4-Amino-N,N-(ethyl,β-acetylaminoethyl)anilin, 4-Amino-N-(β-methoxyethyl)anilin, 3-Methyl-4-amino-N,N-(ethyl,β-acetylaminoethyl)anilin, 4-Amino-N,N-(ethyl,β-mesylaminoethyl)anilin, 3-Methyl-4-amino-N,N-(ethyl,β-mesylaminoethyl)anilin, 4-Amino-N,N-(ethyl,β-sulfoethyl)anilin, 3-Methyl-4-amino-N,N-(ethyl,β-sulfoethyl)anilin, N-((4'-Amino)phenyl)morpholin, N-((4'-Amino)phenyl)piperidin in Form der freien Base oder von Salzen.

5. Verfahren gemäß Anspruch 2,
dadurch **gekennzeichnet**, daß
die p-Aminophenole ausgewählt sind aus p-Aminophenol, 2-Methyl-4-aminophenol, 3-Methyl-4-aminophenol, 2-Chlor-4-aminophenol, 3-Chlor-4-aminophenol, 2,6-Dimethyl-4-aminophenol, 3,5-Dimethyl-4-aminophenol, 2,3-Dimethyl-4-aminophenol, 2-Hydroxymethyl-4-aminophenol, 2-(β-Hydroxyethyl)-4-aminophenol, 2-Methoxy-4-aminophenol, 3-Methoxy-4-aminophenol, 2,5-Dimethyl-4-aminophenol, 2-Methoxymethyl-4-aminophenol.

6. Verfahren gemäß Anspruch 1,
dadurch **gekennzeichnet**, daß
daß die Oxidationsfarbstoffvorstufen Oxidationsfarbstoffvorstufen vom o-Typ sind, ausgewählt aus o-Aminophenolen und o-Phenylendiaminen.

7. Verfahren gemäß jedem Anspruch 1 bis 6,
dadurch **gekennzeichnet**, daß
das Oxidationsmittel ausgewählt ist aus Wasserstoffperoxid, Harnstoffperoxid, Alkalimetallbromaten, Persalzen.

8. Verfahren gemäß jedem Anspruch 1 bis 7,
dadurch **gekennzeichnet**, daß
der pH der auf die keratinischen Fasern aufgebrachten Zusammensetzung 3 bis 6,9 beträgt.

9. Verfahren gemäß jedem Anspruch 1 bis 8,
dadurch **gekennzeichnet**, daß
die zur Färbung der keratinischen Fasern verwendete Zusammensetzung zusätzlich zum 4-Hydroxyindol-Kuppler weitere Kuppler enthält, ausgewählt aus m-Diphenolen, m-Aminophenolen, m-Phenylendiaminen, m-N-Acylaminophenolen, m-Ureidophenolen, m-Carbalkoxyaminophenolen, α-Naphthol, Kupplern mit einer aktiven Methylengruppe, ausgewählt aus Diketoverbindungen und Pyrazolonen.

10. Verfahren gemäß Anspruch 9,
dadurch **gekennzeichnet**, daß
die Kuppler ausgewählt sind aus: 2,4-Dihydroxyphenoxyethanol, 2,4-Dihydroxyanisol, m-Aminophenol, Resorcin, den Monomethylether von Resorcin, 2-Methylresorcin, Pyrocatechol, 2-Methyl-5-N-(β-hydroxyethyl)aminophenol, 2-Methyl-5-N-(β-mesylaminoethyl)aminophenol, 6-Hydroxybenzomorpholin, 2,4-Diaminoanisol, 2,4-Diaminophenoxyethanol, 6-Aminobenzomorpholin, (2-N-(β-Hydroxyethyl)amino-4-amino)phenoxyethanol, 2-Amino-4-N-(β-hydroxyethyl)aminoanisol, (2,4-Diamino)phenyl-β,γ-dihydroxypropylether, 2,4-Diaminophenoxyethylamin, 1,3-Dimethoxy-2,4-diaminobenzol, 2-Methyl-5-aminophenol, 2,6-Dimethyl-3-aminophenol, 3,4-Methylendioxyphenol und 3,4-Methylendioxyanilin sowie deren Salzen.

11. Verfahren gemäß jedem Anspruch 1 bis 10,
dadurch **gekennzeichnet**, daß
die Zusammensetzung ausserdem Direktfarbstoffe und/oder Oxidationsrapidfarbstoffe enthält.

12. Verfahren gemäß jedem Anspruch 1 bis 11,
dadurch **gekennzeichnet**, daß
die Zusammensetzung anionische, kationische, nicht-ionische, amphotere oberflächenaktive Mittel oder deren Mischungen, Verdickungsmittel, Antioxidantien oder jeden weiteren kosmetisch zulässigen Hilfstoff enthält.

13. Verfahren gemäß jedem Anspruch 1 bis 12,
dadurch **gekennzeichnet**, daß
das zur Färbung geeignete Medium aus Wasser oder einer Mischung aus Wasser und einem Lösungsmittel zusammengesetzt ist, das aus C₂₋₄-Niedrigalkanolen, Glycerin, Glycolen, Glycolethern, aromatischen Alkoholen oder deren Mischungen ausgewählt ist.

14. Mittel zur Färbung keratinischer Fasern und insbesondere der Haare,
dadurch **gekennzeichnet**, daß
es mindestens zwei Bestandteile enthält: einen Bestandteil (A) aus einer Zusammensetzung, enthaltend in einem zur Färbung geeigneten Medium den 4-Hydroxyindol-Kuppler und eine in jedem Anspruch 1 bis 6 definierte Oxidationsfarbstoffvorstufe, sowie einen Bestandteil (B) aus einer Zusammensetzung, enthaltend in einem zur Färbung geeigneten Medium ein Oxidationsmittel, wobei der pH der Bestandteile (A) und (B) so eingestellt ist, daß nach Mischung in Mengenverhältnissen von 90 bis 10% für den Bestandteil (A) und von 10 bis 90% für den Bestandteil (B) die sich ergebende Zusammensetzung einen pH von weniger als 7 aufweist.

15. Mittel gemäß Anspruch 14,
dadurch **gekennzeichnet**, daß
der Bestandteil (A) einen pH von 3 bis 10,5 aufweist.

16. Mittel gemäß Anspruch 14 oder 15,
dadurch **gekennzeichnet**, daß
der Bestandteil (A) in jedem Anspruch 1 bis 6 definierte Oxidationsfarbstoffvorstufen vom p- und/oder o-Typ sowie die in den Ansprüchen 1, 9 oder 10 definierten Kuppler in Mengenverhältnissen von 0,05 bis 7 Gew.%, bezogen auf Gesamtgewicht des Bestandteils (A), enthält.

17. Mittel gemäß jedem Anspruch 14 bis 16,
dadurch **gekennzeichnet**, daß
die Konzentration an 4-Hydroxyindol 0,01 bis 4 Gew.%, bezogen auf Gesamtgewicht des Bestandteils (A), beträgt.

18. Mittel gemäß jedem Anspruch 14 bis 17,
dadurch **gekennzeichnet**, daß
der Bestandteil (A) oberflächenaktive Mittel in Mengenverhältnissen von 0,1 bis 55 Gew.%, Lösungsmittel zusätzlich zum Wasser in Mengenverhältnissen von 0,5 bis 40 Gew.%, Verdickungsmittel in Mengenverhältnissen von 0,1 bis 5 Gew.%, Antioxidantien in Mengenverhältnissen von 0,02 bis 1,5 Gew.% und/oder jeden weiteren kosmetisch zulässigen Hilfstoff enthält.

19. Mittel gemäß jedem Anspruch 14 bis 18,
dadurch **gekennzeichnet**, daß
der Bestandteil (B) einen pH aufweist, der einen Minimalwert von 1 bis einen Wert von weniger als 7 hat.

20. Verfahren zur Färbung keratinischer Fasern und insbesondere der Haare,
dadurch **gekennzeichnet**, daß
es eine erste Stufe umfaßt, wobei man die in jedem Anspruch 14 bis 19 definierten Bestandteile (A) und (B) in getrennter Form aufbewahrt und vor der Aufbringung die Bestandteile (A) und (B) in Mengenverhältnissen von 10 bis 90% für den Bestandteil (A) und von 90 bis 10% für den Bestandteil (B) vermischt, und zwar so, daß man eine Zusammensetzung mit einem pH unterhalb 7 erhält, und wobei man diese Mischung unmittelbar nach der Herstellung auf die keratinischen Fasern aufträgt.

21. Vorrichtung aus mehreren Teilen oder Kit zur Färbung,
dadurch **gekennzeichnet**, daß
sie mindestens 2 Teile enthalten, deren erster Teil den in jedem Anspruch 14 bis 18 definierten Bestandteil (A) und deren zweiter Teil den in jedem Anspruch 14 bis 19 definierten Bestandteil (B) umfassen.

22. Vorrichtung gemäß Anspruch 21,
dadurch **gekennzeichnet**, daß
sie mit einem Element ausgerüstet ist, das es erlaubt, die gewünschte Mischung aus den Bestandteilen (A) und (B) auf die Haare zu entleeren.

23. Verfahren zur Färbung gemäß jedem Anspruch 1 bis 13,
dadurch **gekennzeichnet**, daß
man auf die Haare die Zusammensetzung aufbringt und sie 3 bis 40 Minuten verweilen läßt, die Haare spült und ggf. shampooniert, vor einer erneuten Spülung und Trocknung.

24. Verwendung von 4-Hydroxyindol als Kuppler zur Färbung keratinischer Fasern in saurem Medium, und zwar zusammen mit Oxidationsfarbstoffvorstufen, die einen aromatischen Kern mit 2 Amino- oder 1 Amino- und 1 Hydroxi-Gruppen aufweisen, wobei die beiden Gruppen in p- oder o-Position zueinander vorliegen.

25. Gebrauchsfertige Zusammensetzung zur Färbung keratinischer Fasern zur Verwendung im Verfahren gemäß jedem Anspruch 1 bis 13.

26. Zusammensetzung gemäß Anspruch 25,
enthaltend die Verbindung 4-Hydroxyindol in Mengenverhältnissen von 0,01 bis 3,5 Gew.%, bezogen auf Gesamtgewicht der Zusammensetzung.
